(19) Europäisches Patentamt

European Patent Office

Office européen des brevets

(11) Publication number: **0 040 728**
**A1**

## EUROPEAN PATENT APPLICATION

(21) Application number: **81103473.5**

(22) Date of filing: **07.05.81**

(51) Int. Cl.³: **G 01 N 33/54**

(30) Priority: **19.05.80 SE 8003733**

(43) Date of publication of application: **02.12.81**
Bulletin 81/48

(84) Designated Contracting States: **BE DE FR GB NL SE**

(71) Applicant: **PHARMACIA DIAGNOSTICS AB,**
**Rapsgatan 7, S-754 50 Uppsala (SE)**

(72) Inventor: **Carlsson, Jan Per Erik, Spetsvägen 52,**
**S-752 57 Uppsala (SE)**
Inventor: **Drevin, Häkan Nils Yngve, Funbo, Lövsta,**
**S-755 90 Uppsala (SE)**
Inventor: **Ponterius, Per Helge Gillis, Stabby Allé 6A,**
**S-752 29 Uppsala (SE)**
Inventor: **Axen, Rolf Erik Axel Verner, Simonsbo,**
**S-740 22 Bällinge (SE)**

(74) Representative: **Kraus, Walter, Dr. et al, Patentanwälte**
**Dres. Kraus & Weisert Irmgardstrasse 15,**
**D-8000 München 71 (DE)**

(54) **An improvement in and relating to assaying methods involving biospecific affinity reactions.**

(57) An improvement in methods for assaying a component of a sample taken from blood, plasma, serum, cerebrospinal fluid, saliva or urine from mammals, particularly from human beings, involving biospecific affinity reactions, in which there are used 2-4 reactants, of which reactants one, reactant (I), is labelled with at least one enzymatically active group and is soluble in the aqueous liquid in which the biospecific affinity reaction takes place, and in which one of the remaining reactants is the component to be assayed, whereat the reactants through the mentioned biospecific affinity reactions form a conjugate which is insoluble or can be made insoluble and in which labelled reactant (I) is incorporated, and in which methods the enzymatically active group is determined in labelled reactant (I) not bound to the conjugate as a measurement of the amount of said component in the sample. In accordance with the invention the enzymatically active group is an endohydrolase which is capable of degrading a polysaccharide and which is not present in the mentioned sample from said body fluid; and to the mixture of insoluble conjugate in which labelled reactant (I) is present and labelled reactant (I) not bound to the conjugate and thus being in solution there is added for the purpose of determining the aforementioned endohydrolase as a measure of labelled reactant (I) not bound to the conjugate an insoluble substrate for said endohydrolase based on a polysaccharide and capable of being degraded by the mentioned endohydrolase, by hydrolysis of interior glycocidic linkages present in the molecular chains of the polysaccharide.

ACTORUM AG

# AN IMPROVEMENT IN AND RELATING TO ASSAYING METHODS INVOLVING BIOSPECIFIC AFFINITY REACTIONS

The present invention relates to an improvement in assaying methods for assaying a component in a sample from blood, plasma, serum, cerebrospinal fluid, saliva or urine taken from mammals, and preferably from human beings, involving biospecific affinity reactions, in which there are used from 2 to 4 reactants, one of which, reactant (I), is labelled with at least one enzymatically active group and is soluble in the aqueous liquid in which the biospecific affinity reaction is carried out and one of the remaining reactants is said component, the reactants forming, by means of biospecific affinity reactions, a conjugate which is insoluble, or is made insoluble, and in which labelled reactant (I) is incorporated, and in which assaying methods the enzymatically active group is assayed in labelled reactant (I) not bound to the conjugate, as a measure of the amount of said component in the sample.

In assaying methods of the above mentioned type involving biospecific affinity reactions (for instance immunochemical reactions) a reactant (I) [for instance an immunochemical reactant (I)], which is labelled and is soluble in the aqueous liquid in the presence of which the reaction takes place, is reacted with a reactant (II) which exhibits biospecific affinity to (I) [i.e. (II) is a counterpart of (I) and, for instance, (II) is an immunochemical reactant (II)] and, optionally, with a third reactant (III), which exhibits biospecific affinity to (I) and/or (II) [i.e. (III) is a counterpart of (I) and/or (II) and, for instance, (III) is an immunochemical reactant (III)], and optionally with a fourth reactant (IV), which exhibits biospecific

2

0040728

affinity to one of the other reactants [i.e. (IV) is a counterpart of one of said other reactants (I), (II) and (III), for example (III), and (IV) is for example an immunochemical reactant (IV)] to form a conjugate (or complex as it also is called) in which labelled reactant (I) is incorporated.

By "immunochemical reactant" is meant in this connection immunoglobulins (including modified immunoglobulins, e.g. aggregated, and fragments, e.g. Fab- or Fc-fragments), preferably antibodies, and antigens and haptens.

Examples of reactants (I) and reactants (II) [as well as reactants (III) and (IV)] which exhibit biospecific affinity to one another (i.e. they are counterparts of each other) include antigens (or haptens) and specific antibodies directed thereagainst. Other examples include a) Protein A (from S. aureus) and fragments thereof which can bind the Fc-part of immunoglobulins belonging to the IgG-class; b) C1q, which can, for example, bind to heat-aggregated IgG; c) lectins (e.g. Concanavalin A) which, e.g. can bind to specific carbohydrate structures in for example, biopolymers; d) enzyme inhibitors which can bind to their enzyme; e) receptors and ligands; f) physiologically or pharmaceutically active substances capable of binding to corresponding receptors. There are many other such examples of pairs of substances which exhibit biospecific affinity to one another within the biochemical field, e.g. biotin-avidin, intrinsic factor-vitamin B12, etc.

Examples of reactant (III), when such a reactant takes part in the reaction, include an unlabelled reactant (I), for instance for competition with labelled reactant (I), or an antibody directed against antibody or antigen in an antigen-antibody-complex in which one of the components is labelled.

Also a fourth reactant (IV) can take part, for instance in the sequence antigen ← antibody(A) ← antibody(B) → labelled antibody(A).

3

Many other pairs of reactants of biological origin may be mentioned, the interaction of which is used for assaying methods of the above mentioned type, the concentration of one of the participating unlabelled reactants being determined.

A large number of assay methods of the aforementioned basic type, primarily concerning immunochemical assay methods, are known to the art.

To enable the analytically indicatable atom or group in the conjugate or in the labelled reactant (I) which is not bound to the conjugate to be assayed, the conjugate and the labelled reactant (I) not bound to the conjugate are separated from each other, for instance by means of precipitation methods, chromatographic methods (such as gel filtration), or electrophoretic methods.

An example of such a precipitation method is the so-called double-antibody method in which an insoluble immunochemical conjugate is formed which can be separated from components remaining in the solution.

An example of chromatographic methods is the separation of formed soluble conjugate from free (i.e. not bound to the conjugate) labelled reactant (I) by means of gel permeation chromatography.

In recent years said separation has often been achieved by the binding of one of the participating reactants [although not the labelled reactant (I)] to an insoluble polymer, so that the conjugate formed by the biospecific reactions will be attached to the insoluble polymer and can therefore be separated from labelled reactant (I) not bound to the conjugate and thus being present in solution. According to one group of such methods, there is used a water-insoluble polymer material to which there is bound an antibody or an antigen, for example a polypeptide-containing antigen or some other counterpart. Thus, it is known from, for example, British Patent Specifications 1 192 784,

0040728

1 248 764 and 1 248 765 and Biochem. Biophys. Acta 130 (1966) page 257, and Radio-immunoassay Methods (Editors: K E Kirkham and W M Hunter, Churchil Livingstone, London 1971) e.g. pages 405-412 of the article "Solid Phase Antigen Antibody Systems" by L Wide, to use a water-insoluble polymer material to which an antibody or an antigen is bound by bonds of a covalent nature. Further, the US Patent Specification No. 3 646 346 teaches an immunochemical assay method in which there is used antibodies adsorbed on the inner surface of a plastics test tube.

It is also known, when carrying out the immunochemical and analogous assay methods in question, to label one of the reactants [reactant (I)] involved in the assay method with an analytically indicatable atom or group, e.g. an enzymatically active group.

The labelling of the reactant (I) (e.g. an antigen, an antibody etc.) with an enzymatically active group is nowadays well known, and well established techniques herefor are generally known. In this connection it is known that the label can be directly bound to the reactant (I) or that a bridge is introduced between the reactant (I) and the label.

A large number of variants of such assay methods (including immunochemical assay methods as well as analogous assay methods utilizing other reactants than immunochemical reactants which reactants have biospecific affinity to each other) in which there is used a labelled reactant, such as a labelled antigen, a labelled hapten, a labelled antibody or labelled protein A are described in the literature. (See for example the aforementioned references). Thus, for example a) antibodies can be reacted with antigen in a sample and with labelled antigen or b) antibodies can be reacted with antigen in a sample in a manner such that the antigen is bound to the antibody, whereafter there is added labelled antibody which binds to the bound antigen, or c) antigen is reacted with antibody in a sample in a manner such that the antibody binds to the antigen, whereafter there is added labelled antigen which binds to the bound antibody, or d) antigen is reacted with antibody in a sample in a manner such that the antibody binds to the antigen, whereafter there are added labelled antibodies directed against the first-mentioned antibodies and binding thereto, or e) an antigen in a sample is reacted with a labelled antibody, or f) an antibody in a sample is reacted with a labelled hapten or antigen.

The antibodies may belong to one or more immunoglobulin classes. What has been said with respect to assay methods involving antigens and antibodies also applies to analogous assay methods involving other reactants than antigens and antibodies.

It is also well known that such assays are preferably carried out in the presence of an aqueous liquid, e.g. a buffer solution having a suitable pH and ionic strength.

In the case of the quantitative assay of one of the reactants it is also well known to use varying known amounts of this reactant in order to establish standard curves, which are then used to determine unknown amounts of said reactant in a sample.

In order to determine the enzymatically active group in the labelled reactant bound to the conjugate or the labelled reactant not bound thereto, the conventional methods, however, require separation of the two forms of labelled reactant from one another, for example, by centrifugation; siphoning off liquid; slurrying and renewed centrifugation and siphoning off liquid, before a substrate is added to either the conjugate or to the non-bound reactant. These procedural steps are time and labour demanding, require a certain degree of thoroughness and accuracy, necessitate the provision of the requisite equipment and may give rise to error sources.

Consequently, an object of the present invention is to provide a method of the kind set forth in the introduction in which it is not necessary to separate insoluble or insolubilized conjugate from non-bound labelled reactant (I) (which is present in the solution phase) in order to enable the enzymatically active group in the labelled reactant (I) not bound to the conjugate to be determined.

Accordingly there is proposed in accordance with the invention a method which is characterized in that

a) the enzymatically active group is a polysaccharide-degrading endohydrolase which is not present in said sample, and

b) to the mixture of insoluble conjugate in which labelled reactant (I) is present and labelled reactant (I) not bound to the conjugate and thus being in solution, there is added, for determining said endohydrolase as a measure of labelled reactant (I) not bound to the conjugate, an insoluble substrate for said endohydrolase and which is based on a polysaccharide and degraded by said endohydrolase, by hydrolysis of interior glycosidic linkages in the molecular chains of the polysaccharide.

Because the substrate is insoluble, it will be inaccessible to the conjugate-bound enzyme in the insoluble conjugate due to territorial separation.

The conjugate-bound enzyme which is present in the insoluble conjugate and said insoluble substrate cannot come into sufficient contact with one another for the conjugate-bound enzyme to act on the substrate. Thus, the insoluble substrate is inaccessible to the conjugate-bound enzyme and will not therefore react therewith. On the other hand, the insoluble substrate is accessible to the enzymatically active group in the free reactant (I) (present in the solution phase) labelled with enzymatically active group, and the enzymatically active group in said reactant is thus able to react with the substrate.

As will be understood, the insoluble substrate chosen is one which is suitable for assaying the enzyme (endohydrolase) used as the enzymatically active label, said substrate being a substance which is required for effecting an enzymatically catalyzed reaction to determine the enzymatically active labelling group and which shall be in contact with the enzymatically active label. Preferably insoluble substrates are used, which at the enzymatic reaction release substances which are soluble and which are easily determined in the solution by simple measurements. For example, the released substances can be coloured, fluorescent or radioactive.

As before mentioned, said substrate is inaccessible to the conjugate-bound enzyme incorporated in insoluble or insolubilized conjugate, owing to the fact that the substrate selected is one which is insoluble in the aqueous liquid in which the biospecific affinity reactions are carried out, whereat the enzymatically active group in free, labelled reactant (I) present in the solution phase is able to react with the insoluble substrate, while the enzymatically active group in labelled reactant (I) incorporated in the insoluble conjugate does not react with the insoluble substrate. This insoluble substrate may have many different forms. Preferably there are used small insoluble particles, although the substrate may have other forms. For example, the insoluble substrate may be located on the surface of a piece or strip of material, or on the inner surface of a test tube.

The substrate may, for example, be an insoluble substrate previously known with respect to the enzyme in question. An insoluble substrate can be obtained by selecting a soluble polysaccharide based substrate which can be readily coupled to an insoluble carrier. The substrate may, to advantage, be given the form of a water-insoluble, but water-swellable three-dimensional network which is readily accessible to the enzymatically active group in the free labelled reactant (I) in the solution but, on the other hand, not to the enzymatically active group in labelled reactant (I) incorporated in the insoluble conjugate. As an example of such substrates which can be used advantageously in the method according to the invention can be mentioned grains of cross-linked polysaccharides (e.g. dextran) substituted with chromophoric, fluorescent, enzymatic or radioactive groups which can be used when assaying endohydrolases degrading polysaccharides, e.g. dextranase. (See for example US Patent Specification No. 3 676 303).

In principle all insoluble substrates for said endohydrolases which are accessible to the enzymatically active group in labelled reactant (I) in the solution phase but not to the enzymatically active group present in the insoluble conjugate, and corresponding polysaccharide degrading endohydrolases as enzymatically active groups can be used, provided that the enzyme in question is not present in the sample from the body fluids mentioned.

The insoluble substrate is based on a polysaccharide which is degraded by the polysaccharide-degrading endohydrolase used as the enzymatically active labelling group by hydrolysis of interior glycosidic linkages within the molecular chains of the polysaccharide. (The term "based on a polysaccharide" is used here and in the claims to designate a polysaccharide as such as well as a polysaccharide derivative which can be hydrolyzed by an endohydrolase.) Each time a glycosidic linkage is hydrolyzed, a break of the polysaccharide chains occurs and a new reducing endgroup is obtained. Assay methods for the endohydrolase can therefore be based on determination of reducing endgroups. However, other methods based on the breaks of the chains are now preferred in routine analysis. Preferably, therefore,

9

0040728

the substrate used in accordance with the invention comprises a derivative of the polysaccharide, in which derivative there are found indicatable groups or atoms bound by means of covalent bonds, whereat when the endohydrolase in solution reacts with the derivative and hydrolyses interior glycosidic linkages in the derivative, water-soluble fragments of the polysaccharide derivative with attached indicatable groups or atoms are released, whereafter, subsequent to the endohydrolase in solution having been allowed to react with the insoluble substrate for a given length of time, non-dissolved substrate is separated from the liquid containing water-soluble fragments of the reagent dissolved therein, whereafter the indicatable groups or atoms in the liquid or in the undissolved substrate are determined as a measurement of the enzyme activity in the labelled reactant (I) not bound to the conjugate.

In this respect, it is particularly preferred that the substrate comprises a water-insoluble but hydrophilic, swellable three-dimensional network which can be degraded by the endohydrolase, said three-dimensional network comprising molecules of the polysaccharide cross-linked by means of bridges having bonds of a covalent nature, and in which network the indicatable groups or atoms are also bound. In this case, the polysaccharides or derivatives thereof are for example cross-linked by means of bridges which are bound to these molecules by ether bonds, wherein the bridges between the ether bonds may advantageously be straight or branched aliphatic saturated hydrocarbon chains which are substituted by one or more hydroxyl groups (e.g. one to six hydroxyl groups) and which contain 3-30 carbon atoms, preferably 3-20 carbon atoms, and especially 3-10 carbon atoms, and which are optionally broken by one or more oxygen atoms (e.g. one to six oxygen atoms). But of course other types of bridges are also possible, for example ester or amide bound bridges. Examples of such ether-bound cross-linking bridges are

$-CH_2 \cdot CH(OH) \cdot CH_2-$ and $-CH_2 \cdot CH(OH) \cdot CH(OH) \cdot CH-$
and $-CH_2 \cdot CH(OH) \cdot CH_2 \cdot O \cdot CH_2 \cdot CH(OH)-CH_2-$ and
$-CH_2 \cdot CH(OH) \cdot CH_2 \cdot O \cdot (CH_2)_n \cdot O \cdot CH_2 \cdot CH(OH) \cdot CH_2-$ ,

where n is an integer, for example an integer from 2 to 4.

As with previously known assaying methods involving biospecific affinity reactions in which a reactant is labelled with an enzymatically active group in which different enzymes have been used, it is also possible in the method according to the present invention to use different polysaccharide degrading endohydrolases as the enzymatically active labelling groups.

For example, the endohydrolase may be an endodextranase, an endolevanase, an endomannanase, an endopectinase, an endoxylanase, an endoarabinanase, an endoagarase, an endogalactanase or an endochitinase.

In accordance with the invention, there can be chosen as enzyme labelling groups such polysaccharide degrading endohydrolases as those which are not present in the sample from said body fluids.

The enzymatic activity can be measured in a conventional manner, for example by measuring the substance formed by the enzymatic reaction, e.g. by measuring dissolved, chromophoric or fluorescent or radioactive or enzymatically active (i.e. enzymatically active through an enzyme group other than in the labelling group according to the above) conversion products formed, such as coloured soluble fragments when using endodextranase as the labelling group and the aforementioned crosslinked and chromophor-substituted dextran as the substrate.

When a particulate substrate is selected, the particles may have a very small size when so desired, and may be so small that the assay of the enzymatic activity can be determined by turbidity measurements.

The method according to the invention can be applied to particular advantage in competitive systems, although it can also be applied in so-called sandwich systems.

The method according to the invention is preferably applied to those assaying methods which utilize an insoluble carrier material which is inert to the enzymatically active group (e.g. an insoluble polymer such as a synthetic polymer (e.g. plastics) or an insoluble polysaccharide, for example cellulose or agarose or an insoluble product comprising cross-linked polysaccharide, e.g. a polysaccharide cross-linked with epichlorohydrin, provided that the endohydrolase chosen does not react with the polysaccharide material to which one of the reactants [with the exception of labelled reactant (I)] is bound prior to or after establishing the conjugate, or in methods of the double antibody type. When using a reactant bound to a carrier material or when applying the double antibody method, the insoluble conjugate is established in the manner comprehensively described in the literature, see the aforegoing. On the other hand, the covalent binding of the formed conjugate to an insoluble carrier has, as far as is known, not previously been described in the literature, but has been described in patent application No. filed simultaneously herewith.

When applying the method according to the invention to this latter method type there is formed a conjugate of reactants which belong to a system which is soluble in the aqueous liquid, whereafter the formed soluble conjugate is attached with covalent bonds to an insoluble carrier material which is inert against the enzymatically active group, whereafter there is added a substrate which is insoluble in the aqueous liquid, to determine the enzymatically active group in the reactant (I) not bound to the conjugate.

Preferably there is used in this respect a carrier and an unlabelled reactant incorporated in the conjugate which each exhibit a respective type of reactive group, whereat the reactive groups are such which are able to react with one another to form a covelent bond between the carrier and said reactant, and whereat none of the reactive groups is present in any of the other reactants present in the conjugate, and whereat said unlabelled reactant does not compete with labelled reactant (I).

In this way there can be used, for example, a carrier which exhibits pyridyl disulphide groups and an unlabelled reactant which is present in the conjugate and which does not compete with labelled reactant (I) and which exhibits SH-groups or vice versa, which groups are able to react with one another in a thiol-disulfide-exchange reaction.

For more detailed information concerning the introduction of pyridyl disulfide groups or SH-groups in the carrier and reactant respectively, and the reaction of these groups with one another, reference is made to the aforementioned patent application No. filed simultaneously herewith.

In order to increase the sensitivity of the system used in accordance with the invention, there can be used to label the reactant (I) a multiconjugate of a plurality of enzymatically active groups held together by bridges containing splittable covalent bonds, said multiconjugate of enzymatically active groups being in turn bound to reactant (I), preferably with bridges containing splittalbe bonds.

Preferably there is used a disulphide bond -S-S- as the splittable bond.

Splitting is effected with the aid of an insoluble splitting agent which does not have access to the splittable bonds in the multiconjugate of enzymatically active groups present in the conjugate-bound reactant (I) in the insoluble conjugate.

The multiconjugate can be of the kind described in US Patent Specification Serial No. 4 231 999.

The component to be determined in the sample from said body fluids is of course a component which exhibits

0040728

biospecific affinity to a counterpart which is one of the reactants in the biospecific affinity reactions. Thus, e.g. the component is an antigen or hapten and the counterpart is an antibody directed against said antigen or hapten, or the component is an antibody and the counterpart is an antigen or hapten against which the antibody is directed. Thus, the components to be determined are, for example biopolymers, for example polypeptides (the expression polypeptides includes here also proteins which are polypeptides of high molecular weight) including glycopolypeptides and lipopolypeptides. The determination of said components from said body fluids is of great value for diagnostic purposes.

The invention will now be described in more detail with reference to a working example.

Example

Quantitative assay of $\beta_2\mu$-globulin in urine with $\beta_2\mu$-globulin-dextranase derivative in a competitive system

a)    Preparation of $\beta_2\mu$-globulin-dextranase derivative

60 μl N-succinimidyl-3-(2-pyridyldithio)-propionate (SPDP) (from Pharmacia Fine Chemicals AB, Uppsala, Sweden), 5.8 mM in ethanol were added to 3 mg $\beta_2\mu$-globulin dissolved in 0.5 ml of 0.2 M sodium phosphate buffer pH 8.0 while vigorously shaking. Subsequent to allowing the mixture to react for 30 minutes at room temperature, excess reagent and other undesirable low-molecular components were removed by de-salting on a column, 5.5 x 1.6 cm containing particles of dextran cross-linked with epichlorohydrin (Sephadex®G-25 from Pharmacia Fine Chemicals AB, Uppsala) equilibrated with 0.2 M sodium-phosphate buffer pH 8.0. The protein fraction containing $\beta_2\mu$-globulin-2-pyridyldisulphide derivative (2.0 ml) was 86 μM with respect to $\beta_2\mu$-

globulin ($A_{280}$-measurement) and 90 µM with respect to 2-pyridyl disulphide-groups.

100 000 units (1 package) dextranase (endodextranase from Koch-Light Laboratories Ltd., Colnbrook, Bucks, England) were dissolved in 0.2 M sodium phosphate buffer pH 8.0, whereafter the solution was eluted through a Sephadex®G-25 column, 5.5 x 1.6 cm, equilibrated with said buffer. The protein fraction (1.0 ml, $67 \cdot 10^{-6}$ M with respect to the enzyme) was admixed with 70 µl of 5 mM SPDP-solution in ethanol. After 30 minutes, 17 mg of dithiothreitol (from Sigma Chemical Co., Saint Louis, USA) were added. After further 20 minutes excess dithiothreitol was removed from the reaction mixture together with other undesirable low-molecular components, by gel filtration on a Sephadex®G-25 column 5.5 x 1.6 cm, equilibrated with 0.3 M sodium chloride. The protein fraction (2.0 ml) containing thiolated dextranase was 30 µM with respect to protein ($A_{280}$-measurement) and 69 µM with respect to thiol groups.

1.1 ml of 30 µM solution of thiolated dextranase (see above) in 0.3 M NaCl were mixed with 1.0 ml of 86 µM solution of $\beta_2\mu$-globulin-2-pyridyl disulphide in 0.2 M sodium phosphate buffer, pH 8.0 (see above). The reaction was allowed to continue at a temperature of +4°C for 6 calendar days, whereafter 0.8 ml of the reaction mixture was chromatographed on a column, 43 x 1.6 cm, containing cross-linked polyacrylamide gel (Bio-Gel®P 100 from Bio-Rad Laboratories, Richmond, Calif. USA), equilibrated with said buffer. The fractions, which corresponded to a molecular weight in excess of 50 000 Daltons, and which contained $\beta_2\mu$-globulin-dextranase-derivative, were pooled and stored in the presence of 0.02 per cent by weight $NaN_3$ at a temperature of +4°C. When stored in this way it was found that the derivative was stable with respect to its immunochemical and enzymatic activity for at least 3 months.

b)    Preparation of agarose-anti β$_2$μ-globulin derivative
------------------------------------------------------

1.7 grams washed (with 0.2 M NaCl) and CNBr-activated agarose (CNBr-activated Sepharose®4B from Pharmacia Fine Chemicals AB, Uppsala, Sweden), sucked to dryness · were suspended in 3.0 ml of sodium carbonate buffer, pH 9.0, containing 1.7 mg of sheep-antihuman β$_2$μ-globulin-antibodies, whereafter the suspension was rotated for 17 hours at a temperature of +4$^\circ$C. Subsequent to the bonds having been established, the gel was sucked to dryness and residual activated structures were removed by reacting the gel with 3 ml of 0.5 M ethanolamine, pH 9.0, for 30 minutes. The Sepharose®-antibody-derivative was finally washed on a column with 0.3 M NaCl - 0.5 per cent by volume polyoxyethylene sorbitan monolaurate (Tween®20) for 15 hours at a temperature of +4$^\circ$C. Subsequent to being washed, the gel was suspended in 25 ml of washing solution with 0.02 per cent by weight NaN$_3$ added. The concentration of anti β$_2$μ-globulin antibodies was calculated to be 0.4 μM.

c)    Preparation of dyed cross-linked dextran
         (dextranase substrate)
------------------------------------------------

38 grams of dextran cross-linked with epichlorohydrin (Sephadex®G-75 superfine from Pharmacia Fine Chemicals AB, Uppsala, Sweden) was suspended in 680 ml H$_2$O, whereafter 15 grams of reactive blue 2 (from Sigma Chemical Co., Saint Louis, USA) and 3.8 grams of NaOH were added. The colouring process was continued for 4 days at 23$^\circ$C while slowly stirring the mixture. The gel was then washed by repeated sedimentations in water and then shrunk with ethanol. Subsequent to being vacuum dried, the derivative (the substrate) was stored in powder form.

0040728

d)  Determination of the $\beta_2\mu$-globulin content of urine
    with $\beta_2\mu$-globulin-dextranase-derivative in a
    competitive system

9 samples of urine having different contents of $\beta_2\mu$-globulin and a plurality of standard samples of known concentration of $\beta_2\mu$-globulin were analyzed in the following manner. 50 µl of $\beta_2\mu$-globulin dextranase derivative (from a) above), 50 µl Sepharose®4B-anti $\beta_2\mu$-globulin (from b) above) and 100 µl of sample were incubated at a temperature of $23^{\circ}$C while shaking the mixture. The concentration of dextranase substituted $\beta_2\mu$-globulin was $5 \cdot 10^{-9}$M while the concentration of Sepharose®-bound anti $\beta_2\mu$-globulin anti-bodies was $50 \cdot 10^{-9}$M. The enzyme derivative and Sepharose®-suspension was diluted in 0.02 M potassium phosphate buffer, pH 7.4, with 0.3 M KCl, 0.4 per cent by weight casein (from Sigma Chemical Co., Saint Louis, USA), 0.2 per cent by weight human serum albumin (from Sigma Chemical Co., Saint Louis, USA), 0.3 per cent by volume Tween®20 (from Kebo Grave, Stockholm, Sweden) and 0.05 per cent by volume Decon®90 (Decon Laboratories Ltd., Hove, England).

After 90 minutes there were added 200 µl suspension of blue-coloured cross-linked dextran (from c) above) (30 mg/ml) in 0.3 M potassium phosphate buffer, pH 5.5, with 0.4 per cent by weight casein and 0.05 per cent by volume Decon®90 added, whereat the non-bound enzyme derivative was permitted to catalyze the release of soluble fragments of the substrate for 60 minutes at a temperature of $23^{\circ}$C while shaking the mixture, whereafter the reaction was interrupted with 1.0 ml 0.5 M NaOH. As a measurement of the dextranase activity there was used the quantity of solubilized blue-coloured fragments, photometrically measured at 620 nm ($\lambda$ max for the blue dye) in the supernate solutions subsequent to centrifuging.

The $\beta_2\mu$-globulin contents of the various urine samples were determined from a standard curve obtained with the aid of the standard samples. The $\beta_2\mu$-globulin content in all samples was also determined by means of conventional RIA-techniques (RIA = Radio Immuno Assay) (Phadebas® $\beta_2\mu$-test from Pharmacia Diagnostics AB, Uppsala, Sweden), the results of the tests showing good agreement with one another.

## C L A I M S

1. An improvement in assaying methods for assaying a component in a sample from blood, plasma, serum, cerebrospinal fluid, saliva or urine taken from mammals, and preferably from human beings, involving biospecific affinity reactions, in which there are used from 2 to 4 reactants, one of which, reactant (I), is labelled with at least one enzymatically active group and is soluble in the aqueous liquid in which the biospecific affinity reaction is carried out and one of the remaining reactants is said component, the reactants forming, by means of biospecific affinity reactions, a conjugate which is insoluble, or is made insoluble, and in which labelled reactant (I) is incorporated, and in which assaying methods the enzymatically active group is assayed in labelled reactant (I) not bound to the conjugate, as a measure of the amount of said component in the sample, c h a r a c t e r i z e d   i n   that

a)   the enzymatically active group is a polysaccharide-degrading endohydrolase which is not present in said sample, and

b)   to the mixture of insoluble conjugate in which labelled reactant (I) is present and labelled reactant (I) not bound to the conjugate and thus being in solution, there is added, for determining said endohydrolase as a measure of labelled reactant (I) not bound to the conjugate, an insoluble substrate for said endohydrolase and which is based on a polysaccharide and degraded by said endohydrolase, by hydrolysis of interior glycosidic linkages in the molecular chains of the polysaccharide.

2. A method according to claim 1, c h a r a c - t e r i z e d   i n   that the insoluble substrate comprises a derivative of the polysaccharide, in which derivative indicatable groups or atoms are bound by means of covalent bonds, whereat when the endohydrolase in solution reacts with the derivative and hydrolyses glycocidic linkages within the derivative, water-soluble fragments of the polysaccharide derivative with attached indicatable groups or atoms are released, whereafter, subsequent to the endohydrolase in solution reacting with the substrate for a given length of time, undissolved substrate is separated from the liquid with water-soluble fragments of the reagent dissolved therein, whereafter the indicatable groups or atoms are assayed in the liquid or in the undissolved substrate as a measurement of the enzyme activity in the labelled reactant (I) not bound to the conjugate.

3. A method according to claim 2, c h a r a c - t e r i z e d   i n   that the substrate comprises a water-insoluble but hydrophilic, swellable three- dimensional network capable of being degraded by the endohydrolase and comprising polysaccharide molecules cross-linked with bridges having bonds of a covalent nature, in which network said indicatable groups or atoms are also bound.

## European Patent Office

## EUROPEAN SEARCH REPORT

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| D | US - A - 3 676 303 (G.A. BJÖRN et al.) <br><br> * The whole document * <br><br> --- <br><br> FR - A - 2 382 696 (PHARMACIA DIAG- NOSTICS A.B.) <br><br> * Page 1, lines 1-15; page 7, lines 11-21; page 16, line 31 to page 17, line 1; claims 1 and 2 * <br><br> --- | 1-3 <br><br><br><br> 1-3 | G 01 N 33/54 |
| D/P | US - A - 4 231 999 (J.P.E. CARLSSON et al.) <br><br> * Column 1, lines 6-23; column 4, lines 22-28; column 10, lines 6- 11; claims 1-6 *. <br><br> --- | 1-3 | TECHNICAL FIELDS SEARCHED (Int. Cl.³) <br><br> G 01 N 33/54 <br> 33/56 <br> 33/58 <br> 33/68 <br> 33/74 <br> 33/76 <br> 33/78 <br> 33/94 |
| A | US - A - 3 839 153 (A.H.W.M. SCHUURS et al.) <br><br> ---------- | | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 27.08.1981 | GRIFFITH |

EPO Form 1503.1 06.78